# EUROPEAN PATENT APPLICATION

(11) **EP 2 249 160 A1**
(43) Date of publication of application: **10.11.2010**
(21) Application number: 09305359.3
(22) Date of filing: 27.04.2009
(51) Int. Cl.: G01N 33/574, G01N 33/50

(54) **VDAC3-S as a cell marker**

(71) Applicant: Centre National de la Recherche Scientifique, 75016 Paris Cedex 16 (FR)
(72) Inventor: Mazure, Nathalie, 06690 Tourette Levens (FR)
(74) Representative: Le Coupanec, Pascale A.M.P.

(57) **Abstract**

The instant invention relates to the use of VDAC3-S as a cell marker of apoptosis-resistance phenotype of hypoxic cancer cells.

## Description

The instant invention relates to the identification of novel cell markers of apoptosis-resistance phenotype of hypoxic cancer cells and their uses as targets in cancer diagnosis and cancer treatment. More particularly, the present invention relates to methods for evaluating the apoptosis-resistance phenotype of hypoxic cancer cells and for assessing resistance of a cancer to a cancer treatment in a subject. The instant invention also relates to methods for screening candidate active agents useful for restoring apoptosis sensitivity into hypoxic cancer cells and for cancer treatment.

The resistance of cancer cells to chemotherapy has been widely attributed to the hypoxic nature of tumours (Bertout et al., Nat Rev Cancer, 2008, 8:967). Because of the poor vascularization and oxygenation of the internal parts of growing tumours, the ATP production in tumour cells relies upon glycolytic metabolism instead of essentially mitochondrial oxidative respiration. The mechanisms behind this switch in metabolism implicate oncogenic activation, in particular Myc, and the transcription factor hypoxia-inducible factor (HIF), which under limiting oxygen conditions, promotes glycolysis by up-regulating glycolytic enzymes and restricts mitochondrial respiration by up regulating pyruvate dehydrogenase kinase 1, an inhibitor of pyruvate dehydrogenase that drives pyruvate toward mitochondrial oxidative phosphorylation. Thus, hypoxia promotes cell survival through maintenance of ATP production and blocks production of cytotoxic reactive oxygen species formed during mitochondrial oxidative phosphorylation.

Also, mitochondria is known to play an important role in the programmed cell death processes or apoptosis in particular through the release of cytochrome c, from the inter membrane space of mitochondria to the cytoplasm. Mitochondrial dynamics, fusion and fission also participate to apoptosis (Oberst et al., Cell Death Differ, 2008, 15:1139). The proteins optic atrophy 1 (OPAl), mitofusin (Mfn) 1 and 2 regulate fusion whereas dynamin-related protein 1 (Drp1) and Fis1 mediate mitochondrial fission. Mitochondrial fission is an early event in apoptosis, in which Drp1 is recruited with Bax to the outer membrane of mitochondria (OMM) just before cytochrome c release. However, a recent study has established that Bax and Bak can bind Mfn1 and activate Mfn2 thereby directly participating in fusion (Gazaryan et al., Neurochem Res, 2007, 32:917).

The understanding of how hypoxic cancer cells become resistant to chemotherapy appears to be essential for improving and combating cancer. Yet, the mechanisms behind chemotherapy resistance in hypoxic tumours are largely unknown.

Therefore, there is a need for cell markers useful for characterizing the apoptosis-resistance phenotype of hypoxic cancer cells.

There is also a need for a simple and reliable method for identifying apoptosis-resistant hypoxic cancer cells.

There is also a need for treatments allowing to prevent, reduce or suppress the resistance of cancer cells to apoptosis-inducing agents.

There is also a need for the identification of novel anticancer active agents to which cancer cells and tumours do not become resistant.

There is also a need for a method allowing to assess resistance of a cancer to a cancer treatment in a subject.

The present invention has for object to meet these needs.

According to one of its first aspects, the instant invention relates to a use of VDAC3-S as a cell marker of apoptosis-resistance phenotype of hypoxic cancer cells.

Surprisingly, the inventors have observed that the expression of a short isoform of the voltage-dependent anion channel 3 VDAC3 (VDAC3-S) was induced into hypoxic cancer cells and correlated with the change of morphology of mitochondria in a limiting oxygen microenvironment or hypoxia and with the apoptosis-resistance phenotype of cancer cells. The hypoxic cancer cells were observed to be resistant to the staurosporine or etoposide induced-apoptosis, whereas the silencing of VDAC3-S was observed to restore the sensitivity of hypoxic cancerous cells to theses apoptotic agents.

According to an embodiment, a presence, or an increased level of expression and/or activity of VDAC3-S is indicative of an apoptosis-resistance phenotype.

According to another embodiment, a use of VDAC3-S according to the invention, and in particular a presence or an increased level of expression and/or activity of VDAC3-S may be associated with a presence of mitochondria of enlarged phenotype.

According to another embodiment, the instant invention relates to the use of mitochondria of enlarged phenotype as a cell marker of apoptosis-resistance phenotype of hypoxic cancer cells.

In accordance with the invention, the expression "cell marker" is intended to refer to a constituent of a cell or a tissue which may be determined directly or indirectly, for example by direct visualisation or after isolation and labeling with a fluorescent or an isotopic tag, and which is indicative of a physiological, biochemical or morphological state of said cell or tissue.

Within the invention, "VDAC3-S" intends to refer to polynucleotides or polypeptides relating to and representative of VDAC3-S.

Within the invention, the term "phenotype" is intended to refer to the morphological, biochemical and physiological characteristics of a tissue, a cell or a cell organelle.

In accordance with the invention, the expression "apoptosis-resistance phenotype" is intended to refer to a phenotype of a cell or a tissue which is or becomes resistant to at least one agent, chemical or physical, known to cause apoptosis.

Within the invention, the term "hypoxic" intends to relate a condition where the oxygen is depleted or is in a subnormal concentration. For example, hypoxic cell culture conditions may be obtained by subjecting cultured cells to an atmosphere of 1% O₂, 94% nitrogen and 5% CO₂ for at least 48 hours.

Preferably, within the invention, hypoxic conditions refer to conditions where a depletion or subnormal concentration of oxygen is maintained for at least 48 hours, and more preferably for at least 72 hours.

According to another of its aspects, the present invention relates to a method for determining an apoptosis-resistance phenotype of at least one isolated hypoxic cancer cell suspected to be apoptosis-resistant comprising at least the steps of:
a) determining in said hypoxic cancer cell a presence or an absence, or a level of expression and/or activity of VDAC3-S, and
b) comparing said determined presence or absence, or level of expression and/or activity to a control value or a range.

Within the invention, the term "to determine" is intended to refer to a qualitative and/or a quantitative detection.

Within the invention, the expression "control value or range" intends to refer to a value or a range of values of a parameter determined under basal conditions.

For example, with respect to the level of expression of VDAC3-S, the control value or range may be a value or range of values corresponding to a level of expression of VDAC3-S in a cancer cell maintained in normoxia when influence of oxygen concentration is assayed. Alternatively, it may be a level of expression of VDAC3-S in a hypoxic apoptosis-resistant cancer cell without a candidate active anticancer agent when said candidate active anticancer agent is to be assayed.

Typically, for a method of diagnosing, a control value or range may be obtained by determining VDAC3-S in a set of normal subjects not having a cancer. Preferably the control is matched to the patient with respect to some attribute(s) (e.g. age). Depending upon control value or range and the difference between the determined and control value or range, the patient can be diagnosed as having or not apoptosis-resistant cancer cells or a cancer resistant or not to a cancer treatment.

Measurement of the control value or range need not be made contemporaneously, it may be a historical measurement.

According to another aspect, the instant invention relates to a method of assessing a resistance of a cancer to a cancer treatment in a subject, the method comprising at least a step of comparing a presence or an absence, or a level of expression and/or activity of VDAC3-S in a first biological sample obtained from the subject before the treatment has been administered to the subject, and the presence or absence, or level of expression and/or activity of VDAC3-S in a second biological sample obtained from the subject after the treatment has been administered to the subject, wherein a change in the presence or absence, or level of expression and/or activity of VDAC3-S in the second sample relative to the first sample is an indication on the resistance of a cancer to said cancer treatment.

Within the invention, a "cancer treatment" intends to refer to a treatment presented to reduce, suppress, prevent and/or cure a cancer disease, and to cause apoptosis to cancer cells.

According to another aspect, the instant invention relates to a method for screening candidate active agents able to restore apoptosis-sensitivity to an apoptosis-resistant hypoxic cancer cell comprising at least the steps of:
a) providing at least one isolated cell able to express VDAC3-S or a non-human transgenic animal able to express VDAC3-S in conditions suitable for the expression of VDAC3-S,
b) contacting said isolated cell or said non-human transgenic animal with at least one candidate active agent to be screened,
c) determining in said isolated cell or in said non-human transgenic animal a presence or an absence, or a level of expression and/or activity of VDAC3-S, and
d) comparing said determined presence or absence, or level of expression and/or activity to a control value or range.

According to another aspect, the instant invention relates to a method for screening active anticancer agents comprising at the least the steps of:
a) subjecting at least one candidate active anticancer agent known as having anticancer activity to a method of the invention for screening candidate active agents, as previously defined, and
b) selecting the candidate active anticancer agent able to reduce, suppress and/or prevent a presence, or an expression and/or an activity of VDAC3-S.

Within the invention, the expression "anticancer activity" refers to a property of a physical, chemical or biological agent to reduce, suppress and/or prevent the abnormal proliferation and/or invasion of cells *in vivo* or *in vitro,* by inducing apoptosis.

According to another of its aspect, the instant invention relates to a use of at least one nucleic acid sequence able to reduce and/or suppress and/or prevent the presence, or the level of expression and/or activity of VDAC3-S for manufacturing a pharmaceutical composition for restoring sensitivity of a hypoxic apoptosis-resistant cancer cell to an apoptosis-inducing agent for the treatment of cancer.

Within the invention, the term "prevent" intends to refer to a total or partial suppression of a risk of occurrence of an event. When the suppression is partial, the risk of occurrence of a given event is lower than before implementing the instant invention.

According to one embodiment, a cancer considered in the instant invention may be chosen from breast cancer, head-and-neck cancer, liver cancer, ovarian cancer, pancreatic cancer, prostate cancer, renal cancer, skin cancer, testis cancer, lung cancer, cervical cancer, or brain cancer.

According to one of its advantage, the instant invention may be useful in the diagnosis of patient resistant to an anticancer treatment and to provide to said diagnosed patient a more efficient treatment.

According to another of its advantage, the instant invention allows the identification of novel active anticancer agents for the treatment of cancer and the prevention, the reduction and/or the suppression of the apoptosis resistant phenotype of cancer cells.

According to another of its advantage, the instant invention allows to tailor the cancer treatment of a patient by following the risk of occurrence of apoptosis-resistant cancer cells and to provide an opportunity to switch in time to a cancer treatment allowing to reduce, suppress or prevent such risk.

The present invention relates to the identification and the use of a short isoform of voltage dependent anion channel 3 (VDAC3-S) as a novel cell marker useful for characterizing an apoptosis-resistance phenotype of hypoxic cancer cells.

The invention also relates to the determination of the presence or absence, or the level of expression and/or activity of VDAC3-S for the diagnosis, typed and staging of tumours, and in particular of apoptosis-resistant phenotype of cancer cells.

VDAC3 belongs to a group of mitochondrial membrane channels involved in translocation of adenine nucleotides through the outer membrane. VDACs are small, functionally conserved proteins found in the outer mitochondrial membrane of all eukaryotes. These channels may also function as a mitochondrial binding site for hexokinase and glycerol kinase. VDAC3 appears to play a role in the maintenance of the mitochondrial transmembrane potential.

VDAC3 exists as two isoforms, VDAC3-L which is a long isoform and is represented by the polypeptide as set forth in SEQ ID NO: 1, and VDAC3-S which is a short isoform and is represented by the polypeptide as set forth in SEQ ID NO: 2.

VDAC3-S results from an alternative splicing giving rise to an isoform deleted from 38/39 amino acids from the N-terminus part (Sampson et al., J. Biol. Chem., 1998, 273:30482) and having a molecular weight shorter of 4 kDa than VDAC3-L, as observed by Western-blot.

Within the invention, the term "VDAC3" is intended to refer to the long and short isoforms together, whereas VDAC3-L and VDAC3-S are intended to refer, respectively, on the one hand, to the long isoform, and on the other hand, to the short isoform.

VDAC3-L appears to be constitutively expressed in cells whereas VDAC3-S appears to be expressed, as observed by the inventors, upon induction after at least 48 hours, and more preferably after 72 hours of hypoxic condition.

Given that VDAC3-L is constitutively expressed and that VDAC3-S is expressed upon induction, one may understand that the determination of VDAC3-S need not to be specifically operated on VDAC3-S itself, but may be operated on VDAC3 constituted of VDAC3-L and VDAC3-S. An observed difference between the determinations from two samples will be then informative on VDAC3-S.

According to one embodiment, the cell marker VDAC3-S may be human VDCA3-S.

According to another embodiment, the cell marker VDAC3-S is a polynucleotide or a polypeptide.

According to another embodiment of the invention, the cell marker VDAC3-S may be represented by a polynucleotide encoding a polypeptide as set forth in SEQ ID NO: 2, an analogue thereof having an identity of sequence of at least 90%, and more preferably of at least 95%, or a fragment thereof encoding for a polypeptide having at least 8 contiguous amino acids of a polypeptide as set forth in SEQ ID NO: 2.

According to another embodiment, the cell marker VDAC3-S may be represented by a polypeptide as set forth in SEQ ID NO: 2, an analogue thereof having an identity of sequence of at least 90%, and more preferably of at least 95%, and a similar biology activity, or a fragment thereof having at least 8 contiguous amino acids of a polypeptide as set forth in SEQ ID NO: 2.

An identity of sequence of two polynucleotides or two polypeptides may be visually determined or may be determined by any methods usually implemented in the art, such as a BLAST algorithm implemented software available on Pubmed website (www.ncbi.nlm.nih.gov).

A fragment of a polypeptide of the invention may comprise at least 10 contiguous amino acids residues, at least 20 contiguous amino acids residues or at least 30 contiguous amino acids residues of a sequence of the polypeptide as set forth in SEQ ID NO: 2.

According to the invention, a biological activity similar to VDAC3-S with respect to a fragment or to an analogue thereof intends to refer to an activity of maintenance of the mitochondrial transmembrane potential in hypoxia condition and/or the switching of the tubular mitochondria phenotype toward enlarged mitochondria phenotype and/or the resistance of hypoxic cancer cells to apoptosis-inducing agent.

The determination of VDAC3-S may be carried out in an isolated biological sample, and in particular in an isolated cell sample.

As used herein, the term "biological sample" includes a sample from any body fluid or tissue (e.g. serum, plasma, blood, cerebrospinal fluid, urine, kidney tissue) liable to contain VDAC3-S or cancer cells.

An isolated biological sample or an isolated cell sample may be a biopsy, preferably taken from a tumour.

In one embodiment, an isolated biological sample or an isolated cell sample may be obtained from a subject suspected of having a cancer resisting to a cancer treatment.

VDAC3-S is referred to as differential expressed in one sample as compared to another sample when a method used for determining VDAC3-S provides a difference on the presence or absence, or on a level of expression or activity of VDAC 3-S when applied to the two samples.

VDAC3-S is referred to as increased in a first sample if the method for determining VDAC3-S indicates that the level of expression or activity of VDAC3-S is higher in the first sample than in a second sample, or if VDAC3-S is detectable in the first sample but not in the second sample.

Conversely, VDAC3-S is referred to as decreased in the first sample if the method for determining VDAC3-S indicates that the level of expression or activity of VDAC3-S is lower in the first sample than in the second sample, or if VDAC3-S is detectable in the second sample but not in the first sample.

As detailed in the following examples illustrating the invention, expression of VDAC3-S allows the mitochondrial transmembrane potential of cancer cells to be maintained in hypoxic conditions and to modify the morphology of tubular mitochondria toward enlarged mitochondria phenotype.

According to one embodiment, the use of VDAC3-S according to the invention may be associated with the determination of the presence or the absence of mitochondria of enlarged phenotype.

In particular, a presence, or an increased level of expression and/or activity of VDAC3-S may be associated with a presence of mitochondria of enlarged phenotype.

Mitochondria of enlarged phenotype present an increased size with respect to mitochondria of tubular phenotype which are usually observed in normal and healthy cells.

The determination of the presence or absence of mitochondria of enlarged phenotype may be carried out by any methods of cell biology or cell imaging. For example, one may use direct observation with electron microscopy or fluorescent microscopy after immunolabeling mitochondria with antibodies, for example directed to cytochrome c, tagged with a fluorescent or a chemiluminescent molecule or an enzyme able to convert colorimetric substrate.

The present invention relates to a method for determining an apoptosis-resistance phenotype of at least one isolated hypoxic cancer cell suspected to be apoptosis-resistant comprising at least the steps of:
a) determining in said hypoxic cancer cell a presence or absence, or a level of expression and/or activity of VDAC3-S, and
b) comparing said determined presence or absence, or level of expression and/or activity to a control value or range.

According to one embodiment, a presence, or an increase of said level of expression and/or activity relative to the control value or range detects an apoptosis-resistance phenotype.

According to another embodiment, a method of the invention may further comprise a step of determining an absence or a presence of mitochondria of enlarged phenotype.

The determination of the presence or absence, level of expression and/or activity of VDAC3-S, may be carried out by any conventional technique of cell biology, analytical biochemistry, molecular biology or recombinant DNA techniques within the art useful for evaluating or quantifying the presence or absence, or level of expression or activity of a cell constituent such as a polypeptide or a polynucleotide.

The determination may be operated directly on a sample or after extracting VDAC3-S from a sample.

According to one embodiment, the determination in step a) may be carried out by a method chosen from mass spectrometry such as LC-MS, GC-MS, chromatographic separations such as one or 2-D gel separations, binding assays such as immunoassays or hybridization, enzyme assays such as ELISA, or competitive inhibition assays.

Preferably, step a) may be carried out by a method chosen from Western-blot, immunoassays ELISA, fluorescence microscopy, flow cytometry, direct sequencing, gel electrophoresis, column chromatography, Northern or Southern blot, PCR, RT-PCR or quantitative PCR.

A presence or an increase of the level of expression and/or activity of VDAC3-S relative to the control value or range detects an apoptosis-resistance phenotype.

According to an embodiment, the determination of presence or absence, or level of expression and/or activity of a polynucleotide relating to VDAC3-S, such as RNA or DNA, may be carried out by any method known in the art.

Methods for polynucleotide determination include direct sequencing, gel electrophoresis, column chromatography, Northern or Southern-blot, PCR, RT-PCR or quantitative PCR.

Hypoxic cancer cells expression VDAC3-S may be used as a source of mRNA, which may be assayed directly or reverse transcript into cDNA for analysis. The nucleic acid may be amplified by conventional techniques, such as the polymerase chain reaction (PCR) or reverse transcriptase PCR (RT-PCR) to provide sufficient amount for analysis.

The amplified nucleotides may then be subjected to gel electrophoresis and then stained with a nucleic acid dye.

Alternatively, a detectable label may be included in an amplification reaction. Suitable labels include fluorochromes, such as fluorescein isothiocyanate, rhodamine or Texas Red^{®}. The label may be a two stage systems, where the amplified polynucleotide is conjugated to biotin, haptens, etc, having a high affinity binding partner, e.g. avidin, specific antibodies, etc, where the binding partner is conjugated to a detectable label. The label may be conjugated to one or both of the primers.

Alternatively, the pool of nucleotides used in the amplification may be labelled so as to incorporate the label into the amplification product. The sample polynucleotide may be analyzed by any methods known in the art.

According to another embodiment, a polynucleotide determination may include polynucleotide extraction from an isolated biological sample or isolated cell sample, followed by hybridization of a labelled probe (e.g., a complementary polynucleotide) specific for the target polynucleotide, and detection of the probe (e.g., Northern blotting).

Detection of the presence or number of copies of a polynucleotide relating to VDAC3-S, may be performed using, for example, Southern or Northern analysis, in which total nucleic acids from a cell or tissue sample is extracted, hybridized with a labelled probe (e.g. a complementary DNA molecule), and the probe is detected. The label group can be a radioisotope, a fluorescent compound, an enzyme, or an enzyme co-factor.

Arrays provide a high-throughput technique that can assay a large number of polynucleotide in a sample. In one embodiment of the invention, an array may be constructed comprising polynucleotides relating to VDAC3-S, which array may further comprised other sequences known to be up- or down-regulated in tumour hypoxic cells. This technology may be used as a tool to test for differential expression.

A variety of methods of producing arrays are known in the art and contemplated for use in the invention.

According to an embodiment, the determination of presence or absence or level of expression and/or activity of a polypeptide relating to VDAC3-S may be carried out by any method known in the art. For example, the determination of a polypeptide relating to VDAC3-S may be based on the functional or antigenic characteristics of the polypeptide. The determination may be operated directly on the sample or after extraction.

Methods for polypeptide determination include immunoassays, Western-blot, ELISA, fluorescence microscopy or flow cytometry.

According to one embodiment, determination may utilize staining of cells or histological sections, performed in accordance with conventional methods.

Alternatively, polypeptide determination may include polypeptide extraction from a cell or tissue sample, followed by binding of a labelled probe (e.g., an antibody) specific for the target polypeptide, and detection of the probe. The label group can be a radioisotope, a fluorescent compound, an enzyme, or an enzyme co-factor.

According to one embodiment, polypeptide determination may be assessed by gel electrophoresis or column chromatography.

In a preferred embodiment, a polypeptide relating to VDAC3 may be determined by one- or two-dimensional gels, optionally comprising a step of transfer on a blot, such as Western blot. Such technique allows the proteins to be separated and further identified and quantified for example by silver staining or antibodies tagged with fluorescence or radioactive label.

Western blot may be advantageously performed on protein gels or protein spots on filters, using detection system specific for polypeptide relating to VDAC3-S, and may conveniently use a labelling method as described for the sandwich assay.

According to another embodiment, a polypeptide relating to VDAC3-S may be determined using antibodies or other specific biding members added to a biological sample or to a cell sample or a lysate thereof and incubated for a period of time sufficient to allow binding to VDAC3-S, for example at least about 10 minutes. Antibodies may be labelled with radioisotope, enzymes, fluorescent molecules, chemiluminescent molecules, or other labels for direct detection. Alternatively, a second stage antibody or reagent may be used to amplify the signal. Such reagents are well-known in the art. For example, a primary antibody may be conjugated to biotin, with horse radish peroxydase-conjugated avidin added as a second stage reagent. Final detection may use a substrate that undergoes a colour change in the presence of the peroxydase. A second stage antibody may also, for example, be labeled with a fluorescent or a radioisotopic molecule. The absence or presence or relative amount of antibody binding may be then determined by various methods, including flow cytometry, fluorescence microscopy, radiography, scintillation counting, etc.

Also a conventional sandwich type assay may be used. For example, a sandwich assay may first attach VDAC3-S specific antibodies to an insoluble surface or support. In such method, a series of standards containing known a concentration of VDAC3-S may be assayed in parallel with the samples to serve as controls. The incubation time should be sufficient for binding, generally from 0.1 to 3 hours is sufficient. After incubation, the insoluble support is generally washed and a solution containing a second antibody is applied, which may be labelled to facilitate direct or indirect detection and/or quantification of binding. Examples of labels include radiolabels such as ³H or ¹²⁵I, fluorescent molecules, dyes, beads, chemiluminescent molecules, colloidal particles, and the like. Examples of labels permitting indirect measurements include enzymes where the substrate may be provided for a coloured or fluorescent product.

In one embodiment, polypeptide relating to VDAC3-S may be determined using a standard immunoassay, such as sandwiched ELISA using matched antibody pairs and fluorescent or chemiluminescent detection. Commercially available or custom monoclonal or polyclonal antibodies may be used.

In some cases, a competitive assay may be used. In such assay, a competitor to VDAC3-S is added to the isolated biological sample or the isolated cell or lysate thereof. The competitor and VDAC3-S compete for binding to a specific binding partner. Usually, the competitor molecule may be labelled and detected as previously described, whereas amount of competitor binding will be proportional to the amount of VDAC3-S presence. The competitor molecule will be from about 10 times the maximum anticipated VDAC3-S concentration to about equal concentration in order to make the most sensitive and linear range of detection.

In some embodiments, the methods may be adapted for use *in vivo,* e.g. to locate or identify sites where apoptosis-resistant hypoxic cancer cells are present.

In these embodiments, a detectably labelled moiety, e.g. an antibody, which is a specific for VDAC3-S is administered to an individual (e.g. by injection), and labelled cells are located using standard imaging techniques, including but not limited to magnetic resonance imaging, computed tomography scanning, and the like. In this matter, apoptosis-resistant hypoxic cancer cells are differentially labelled.

An activity, and in particular a biological activity, relating to VDAC3-S may be determined by determining the presence or the absence of mitochondria with an enlarged phenotype in an isolated biological sample or an isolated cell sample as previously indicated, or the mitochondrial transmembrane potential of isolated hypoxic cells or the resistance to the apoptosis of hypoxic cells.

The mitochondrial transmembrane potential (ΔΨm) may be measured by any conventional techniques known in the art.

For example, the mitochondrial transmembrane potential may be determined by the use of fluorescent molecules which accumulate specifically in the inter membrane space of the mitochondria, such as DiOC₆(3). The fluorescence may be observed by any known fluorescent methods, such as fluorescent microscopy or flow cytometry.

The apoptosis-resistance inducing activity of VDAC3-S may be observed by subjecting isolated cells, and preferably isolated cancer cells, to hypoxic conditions and contacting a first sample of hypoxic cells with an apoptosis-inducing agent, such as etoposide or staurosporine, and comparing the death cells rate or the presence or absence or the level of expression and/or activity of different markers specific to apoptosis in the first sample to a second sample of hypoxic cells not contacted with said agent.

Markers specific to apoptosis, may be, for example, caspase activity, annexin V flip-flop or DNA ladder, and may be detected using any known methods in the art, such as fluorescence microscopy, flow cytometry, ELISA or gel electrophoresis.

The present invention also relates to a method for assessing a resistance of a cancer to a cancer treatment in a subject, the method comprising at least a step of comparing a presence or absence, or a level of expression and/or activity of VDAC3-S in a first biological sample obtained from the subject before the treatment has been administered to the subject, and the presence or absence, or level of expression and/or activity of VDAC3-S in a second biological sample obtained from the subject after the treatment has been administered to the subject, wherein a change in the level of expression and/or activity of VDAC3-S in the second sample relative to the first sample is an indication on a resistance of a cancer to said cancer treatment.

The determination of VDAC3-S may be performed as previously described.

In some embodiments, the presence or absence, or level of expression and/or activity of VDAC3-S may be compared to the presence or absence, or level of expression and/or activity of VDAC3-S in a different tissue or biological "compartment".

As will be apparent to those of ordinary skill in the art, the above description is not limited to making an initial diagnosis of resistant cancer, but also is applicable to confirming a provisional diagnosis of resistant cancer or "ruling out" such a diagnosis.

Furthermore, a presence or an increased level of expression and/or activity of VDAC3-S in a sample obtained from a subject suspected of having a cancer resistant to a cancer treatment, or at risk for developing a cancer resistant to a cancer treatment, is indicative that the subject has or is at risk for developing a resistant cancer.

According to another embodiment, the invention also provides a method for determining a subject's risk of developing a cancer resistant to a cancer treatment, the method comprising obtaining a biological sample from a subject, detecting the presence or absence, or level of expression and/or activity of VDAC3-S in the sample, and comparing the result to the presence or absence, or level of expression and/or activity of VDAC3-S in a sample obtained from a subject having not a cancer resistant to a cancer treatment, or to a control value or range, wherein a presence or an increase of level of expression or activity of VDAC3-S is correlated with the risk of a resistant cancer.

According to another embodiment, the invention also provides a method for determining a stage or severity of a cancer resistant to a cancer treatment.

Such a method may comprise obtaining a biological sample from a subject having a cancer, determining a presence or absence, or level of expression and/or activity of VDAC3-S in the sample, and comparing the result to the presence or absence or level of expression or activity of VDAC3-S in a sample obtained from a subject having not a resistant cancer, or to a control value or range, wherein an increase or decrease of VDAC3-S is correlated with a stage or severity of the disease.

A resistant cancer may be diagnosed in a subject if a level of expression or activity of VDAC3-S in the subject sample is statistically more similar to the level of expression or activity of VDAC3-S that has been associated with a resistant cancer than the level of expression or activity of VDAC3-S that has been associated with the control.

A method of the present invention may be used to make a diagnosis of a resistant cancer, independently from other information such as the subject symptoms or the results of other clinical or paraclinical tests. However, a method of the present invention may be used in conjunction with such other data points.

In another aspect, the invention provides a method for monitoring the progression of a cancer resistance to a cancer treatment in a subject who has a cancer.

Such method may comprise obtaining a first biological sample from a subject having a cancer and subjected to a cancer treatment at time to, determining a presence or absence, or level of expression and/or activity of VDAC3-S in the sample, and comparing the result to a presence or absence, or level of expression and/or activity of VDAC3-S in a second biological sample obtained from said subject at a later time t₁, wherein a presence or absence or an increase or decrease of VDAC3-S is correlated with progression of the cancer resistance to a cancer treatment.

Depending upon the difference between the determinations, it can be seen whether the VDAC3-S is present or absent or whether its level of expression or activity has increased, decreased, or remained constant over time.

A further deviation of VDAC3-S in a direction indicating resistant cancer, or the measurement of additional increased VDAC3-S, would suggest a progression of the cancer resistance to the cancer treatment during the interval.

Subsequent sample acquisitions and determination may be performed as many times as desired over a range of times t₂ to tₙ.

The ability to monitor a subject by making serial VDAC3-S level of expression and/or activity determinations over time may be used to follow a progression of the cancer resistance, to predict exacerbations or to indicate the clinical course of the cancer resistance.

In another aspect, the present invention provides a method for screening candidate active agents for use as therapeutic compounds.

According to one embodiment, the present invention provides a method for screening candidate active agents able to restore apoptosis-sensitivity to an apoptosis-resistant hypoxic cancer cell comprising at least the steps of:
a) providing at least one isolated cell able to express VDAC3-S or a non-human transgenic animal able to express VDAC3-S in condition suitable for the expression of VDAC3-S,
b) contacting said isolated cell or said non-human transgenic animal with at least one candidate active agent to be screened,
c) determining in said isolated cell or in said non-human transgenic animal a presence or absence, or level of expression and/or activity of VDAC3-S, and
d) comparing said determined presence or absence, or level of expression and/or activity to a control value or range.

According to another embodiment, the present invention provides a method for screening candidate active anticancer agents comprising at the least the steps of:
a) subjecting at least one candidate active anticancer agent known as having anticancer activity to the screening method as previously defined, and
c) selecting the active candidate anticancer agent able to reduce, suppress and/or prevent presence or an expression and/or an activity of VDAC3-S.

The present invention provides a method for screening candidate active agents able to bind to, to modulate or to mimic the biological activity of polypeptide or polynucleotide relating to VDAC3-S.

Candidate compounds which affect the presence or absence, or the level of expression and/or activity of VDAC3-S may be identified using any suitable methods or techniques known in the art, and in particular in using the methods previously described.

A screening method of the invention may be performed using an *in vitro* model, an isolated cell, a genetically modified cell or animal.

When performed on isolated cells, a method for screening of the invention may be performed on primary, immortalized or tumour cells in which VDAC3-S is introduced using an inducible expression system.

Transgenic animals or cells derived therefrom may also be used in a screening method of the invention. Transgenic animals may be made by any methods known in the art. For example, transgenic animals may be made through homologous recombination where the normal VDAC3 locus is altered. Alternatively, a nucleic acid construct may be randomly integrated into the genome. Vectors for stable generation include plasmids, retrovirus and other animal viruses, YACS, and the like.

In a further additional embodiment, VDAC3-S may be used to screen candidate active agent, for example, in a clinical trial, to determine whether a candidate active agent is effective in preventing and/or treating a resistant cancer.

At time to, a biological sample is obtained from each subject in population of subjects diagnosed with resistant cancer. Next, assays are performed on each subject's sample to measure a presence or absence, or levels of expression and/or activity of VDAC3-S.

Next, a predetermined dose of a candidate active agent is administered to a portion or sub-population of the same subject population. Active agent administration may follow any suitable schedule over any time period. In some cases, varying doses are administered to different subjects within the sub-population, or the candidate active agent is administered by different routes. At time t₁, after active agent administration, a biological sample is acquired from the sub-population and the same assays are performed on the biological samples as were previously performed to obtain determination of presence, absence or values.

As previously indicated, subsequent samples acquisitions and determinations may be performed as many times as desired over a range of times t₂ to tₙ.

In such a study, a different sub-population of the subject population serves as a control group, to which a placebo is administered. The same procedure is then followed for the control group: obtaining the biological sample, processing the sample, and determining VDAC3-S to obtain a determination chart.

Specific doses, delivery routes, time of administration can also be examined. The method is performed by administering the candidate active agent at specified dose, delivery routes or period of time to subjects with resistant cancer; obtaining biological samples from the subjects; determining the presence or absence, or level of expression and/or activity of VDAC3-S in each of the biological samples; and, comparing the determined presence or absence, or level of expression and/or activity of VDAC3-S for each sample with other samples and/or a control value or range.

Typically, the control value or range may be obtained by determining VDAC3-S in the subject before active agent administration. Depending upon the difference between the determined and control value or range, the dose, route of delivery or time of administration may be considered to have an effect on a resistant cancer.

According to one embodiment, the present invention provides a use of at least one nucleic acid sequence able to reduce and/or suppress and/or prevent the presence or level of expression and/or activity of VDAC3-S for manufacturing a pharmaceutical composition for restoring sensitivity of a hypoxic apoptosis-resistant cancer cell to an apoptosis-inducing agent for the treatment of cancer.

The nucleic acid may be a gene or an antisense sequence able to control, and in particular to down-regulate, the expression of VDAC3-S. Such sequences may, for example, be transcribed in the target cell into RNAs complementary to cellular mRNAs and thereby block their translation into protein, according to the technique described in EP 0 140 308. Alternatively, such sequences may be introduced as such in the cells under the form of RNA.

The nucleic acids may be single- or double-stranded, as well as short oligonucleotides or longer sequences.

These nucleic acids may be of human, animal, plant, bacterial or viral origin and the like. They may be obtained by any technique known in the art, and in particular by screening libraries, by chemical synthesis or alternatively by mixed methods including chemical or enzymatic modification of sequences obtained by screening libraries. They may be chemically modified.

A nucleic acid sequence may be chosen from a siRNA, a miRNA, a shRNA, a hybrid sequence or a synthetic or semi-synthetic sequence of oligonucleotide.

More particularly, antisense molecules may be antisense oligonucleotides (ODN), particularly synthetic ODN having chemical modifications from native nucleic acids or nucleic acid constricts that express such antisense molecule as RNA. Antisense molecules may inhibit gene expression through various mechanisms, e.g. by reducing the amount of mRNA available for translation through activation of RNAse H, or steric hindrance. One or a combination of antisense molecules may be administered where a combination may comprise a multiple different sequences.

Antisense molecules may be produced by expression of all or part of the target gene sequence in an appropriate vector, where the transcriptional initiation is oriented such that an antisense strand is produced as an RNA molecule.

Alternatively, the antisense molecule may be a synthetic oligonucleotide. Antisense oligonucleotides may be chemically synthesized by any methods known in the art. Preferably oligonucleotides are chemically modified from the native phosphodiester structure in order to increase the intracellular stability and binding affinity.

Antisense oligonucleotides may generally comprise at least 7, usually at least 12, more usually at least about 20 nucleotides in length, and not more than about 500, usually not more about 50, more usually not more than about 35 nucleotides in length, where the length is governed by efficiency of inhibition, specificity, including absence of cross-reactivity, and the like.

According to one embodiment the therapeutic nucleic acid may be a siRNA, miRNA or shRNA, and in particular is a siRNA.

Preferably, antisense nucleotides usable for the invention may be siRNA commercially available from Santa Cruz Biotechnology (VDAC3 siRNA (m): sc-42360).

As an alternative to antisense inhibitors, catalytic nucleic acid compounds, e.g. ribozymes, antisense conjugates, etc. may be used to inhibit gene expression.

The therapeutic nucleic acids may comprise sequences encoding ribozymes, which are capable of selectively destroying target RNAs.

According to one embodiment, expression vectors may be used to introduce nucleic acid sequences and, in particular antisense molecules or catalytic nucleic acid compounds.

Expression vectors may comprise a transcription cassette comprising transcription initiation reagent, the target nucleic acid sequence and a transcriptional termination reagent. The transcription cassettes may be introduced into a variety of vectors, e.g. plasmid; retrovirus, e.g. lentivirus; adenovirus; and the like, where the vectors are able to transiently or stably be maintained in the cells, usually for a period of at least about one day, more usually for a period of at least about several days to several weeks.

A pharmaceutical composition of the invention may be formulated with appropriate pharmaceutically acceptable carriers or diluents and may be formulated into preparation in solid, semi-solid, liquid or gaseous forms, such as tablets, capsules, powders, granules, emulsions, suspensions, inhalant, gels, microspheres, and the like.

In one embodiment, a pharmaceutical composition of the invention may be provided as oral dosage forms, such as tablets, capsules (each including timed release and sustained release formulations), pills, powders, granules, elixirs, tinctures, solutions, suspensions, syrups and emulsions.

In another embodiment a pharmaceutical composition of the invention may be provided as injectable forms. The nucleic acid sequences may then be provided as such or formulated with various vehicles, such as liposomes or transfection polymers.

A pharmaceutical composition of the invention may preferably comprise nucleic acid sequences suspended in a pharmaceutical acceptable carrier, for example an aqueous carrier. A variety of aqueous carriers may be used e.g., water, buffered water, 0.4% saline, 0.3% glycine, hyaluronic acid and the like.

A pharmaceutical composition may be sterilized by conventional, well-known sterilization techniques, or may be sterile filtered. The resulting aqueous solutions may be packaged for use as is, or lyophilized, the lyophilized preparation being combined with a sterile solution prior to administration.

A pharmaceutical composition of the invention may contain pharmaceutically acceptable auxiliary substances as required to approximate physiological conditions, such as pH adjusting and buffering agents, tonicity adjusting agents, wetting agents and the like.

Such preparations may also routinely contain pharmaceutically acceptable concentrations of antioxidants, preservatives, compatible carriers, adjuvants, and optionally other therapeutic agents.

A pharmaceutical composition of the invention comprises nucleic acids sequences of the invention in an effective amount.

An effective amount is an amount of nucleic acids sequences that alone, or together with further doses may result in the desired response. An effective amount may depend upon a variety of factors, such as the route for administration, whether the administration is in single or multiple doses, and individual patient parameters including age, physical condition, size, weight, and the stage of the disease. These factors are well known to those of ordinary skill in the art and can be addressed with no more than routine experimentation.

As such, administration of a pharmaceutical composition in the invention may be achieved in various ways, including oral, buccal, rectal, parenteral, intraperitoneal, intradermal, transdermal, intra-tracheal, etc, administration.

A pharmaceutical composition of the invention may preferably be administered by injection or by a mucosal route, or a combination thereof.

Injection route may be, for example, intraperitoneal, intradermal, subcutaneous intravascular or intramuscular route.

Any mucosal route may be used, such as genito-urinary route as for example vaginal route, gastro-intestinal route, anorectal route, respiratory route, upper mucosal tissue, mouth-nasal route and mixtures thereof.

According to another aspect, the instant invention is also directed to a method to prevent, reduce and/or treat cancer resistance comprising at least a step of administration to a subject in need thereof an effective amount of nucleic acid sequences according to the instant invention.

The various aspects of the instant invention will be further illustrated by the following examples, which should not in any case be constructed as limiting the scope of the instant invention.

### LEGENDS OF THE FIGURES

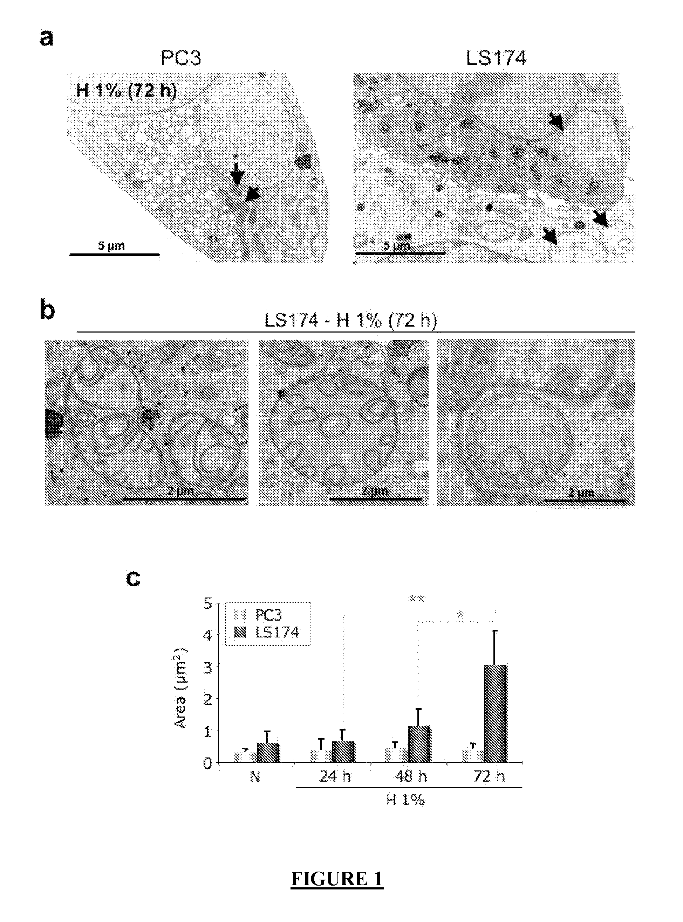

Figure 1 illustrates the formation of enlarged mitochondria in hypoxic cells. Figure 1a shows electron micrographs of PC3 and LS174 cells in hypoxia (H, 1% O₂, 72h). Arrows denote tubular mitochondria in PC3 cells and enlarged mitochondria in LS174 cells. Figure 1b shows electron micrographs of enlarged mitochondria. Figure 1c illustrates area of mitochondria (n=2, 50 mitochondria per experiment, means ± s.d. **P*<0.05, ***P*<0.01) in normoxia (N, 21% O₂ 72h) or hypoxia (H, 1% O₂, 72h).

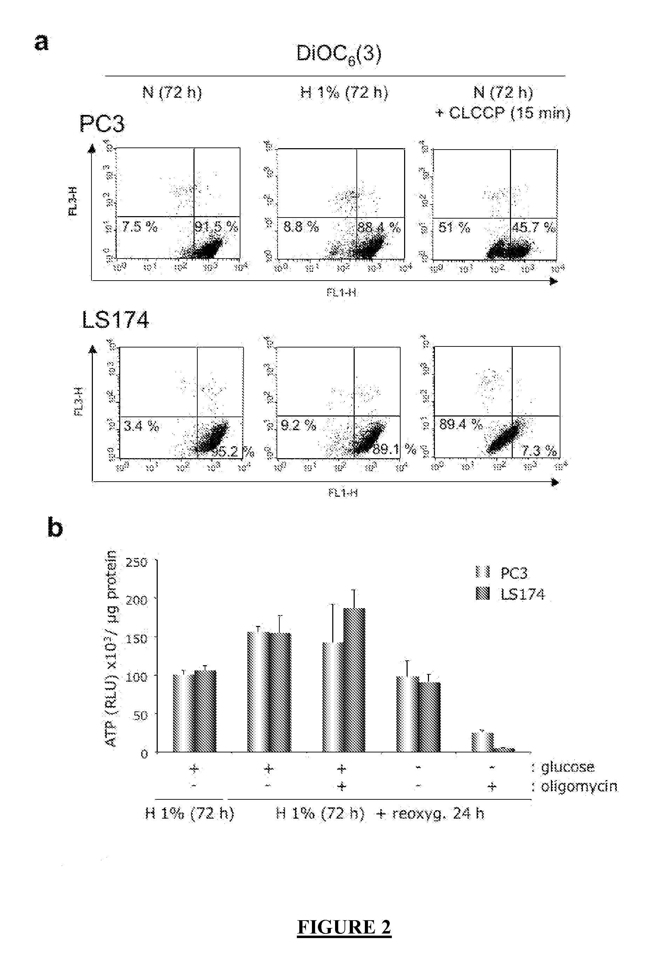

Figure 2 illustrates that enlarged mitochondria are functional. Figure 2a shows results of flow cytofluorimetry of cells, in normoxia (N, 21 % O₂), hypoxia (H, 1% O₂) or normoxia plus a mitochondrial decoupling compound CLCCP, at 72h, stained with DiOC₆(3) (ΔΨm) and propidium iodide (cell viability). The percentage of low and high DiOC₆(3) stained cells, left and right, respectively. Figure 2b shows the ATP levels in cells incubated first in hypoxia (H, 1% O₂, 72h) and then in normoxia without (-) or with (+) glucose or the inhibitor of mitochondrial respiration oligomycin (n=8 of two experiments, means ± s.d. ****P*<0.005).

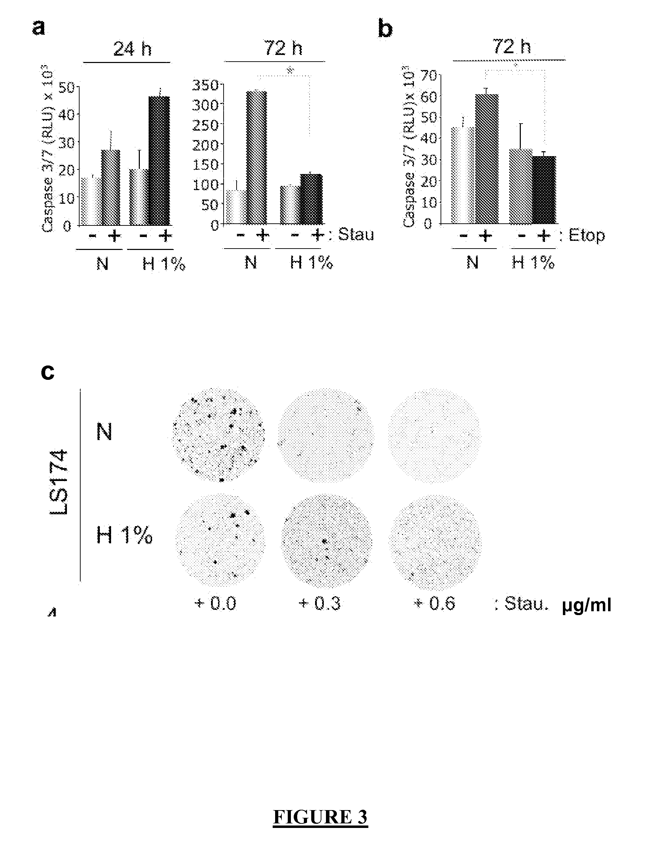

Figure 3 shows that LS 174 cells with enlarged mitochondria are protected from apoptotic stimuli. Figure 3a illustrates the caspase 3/7 activity of cells in normoxia (N, 21 % O₂, 72h) or hypoxia (H, 1% O₂, 72h) without (-) or with (+) staurosporine 1 µM (Stau) at 24h and 72h (means ± s.d. **P* < 0.005). Figure 3b illustrates the caspase 3/7 activity of cells in normoxia (N, 21% O₂) or hypoxia (H, 1% O₂) without (-) or with (+) etoposide 10 µg/ml (Etop) at 72h (means ± s.d. *P < 0.005). Figure 3c shows cells in 100 mm dishes in normoxia (N) or hypoxia (H), maintained for 10 days without or with Stau 0.3 or 0.6 µg/ml and stained for viable colonies.

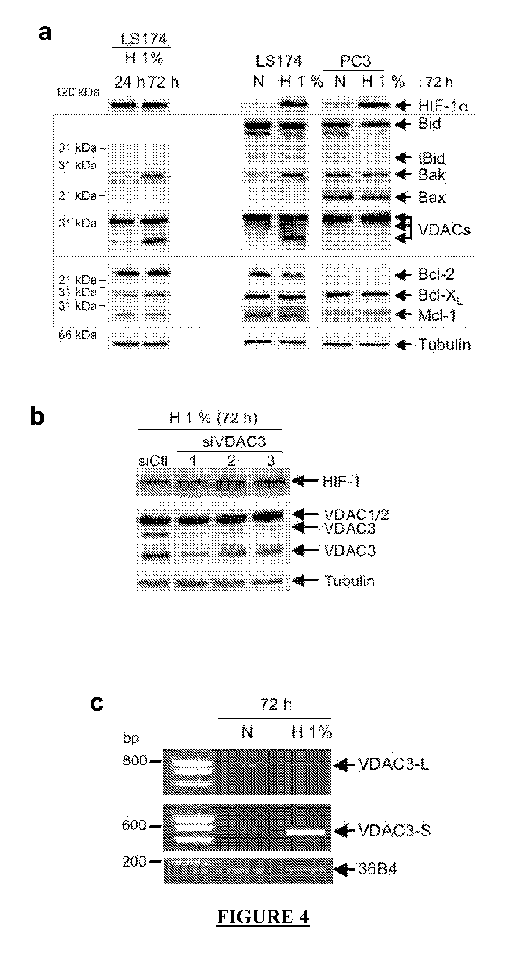

Figure 4 show hypoxia-dependent induction of VDAC3 in LS174. Figure 4a shows immunoblot of the expression of the indicated proteins of hypoxic or normoxic LS174 or PC3 cells at 24h and 72h. Figure 4b shows immunoblot of VDAC1, VDAC2 and VDAC3 in control (+siCt1) or VDAC3 (+siVDAC3) silenced LS174 cells. VDAC3 was normalized to tubulin (3 experiments). Figure 4c shows the specific induction of the iso form VDAC3-S in LS 174 under hypoxic conditions. (H, 1% O₂ for 72h)

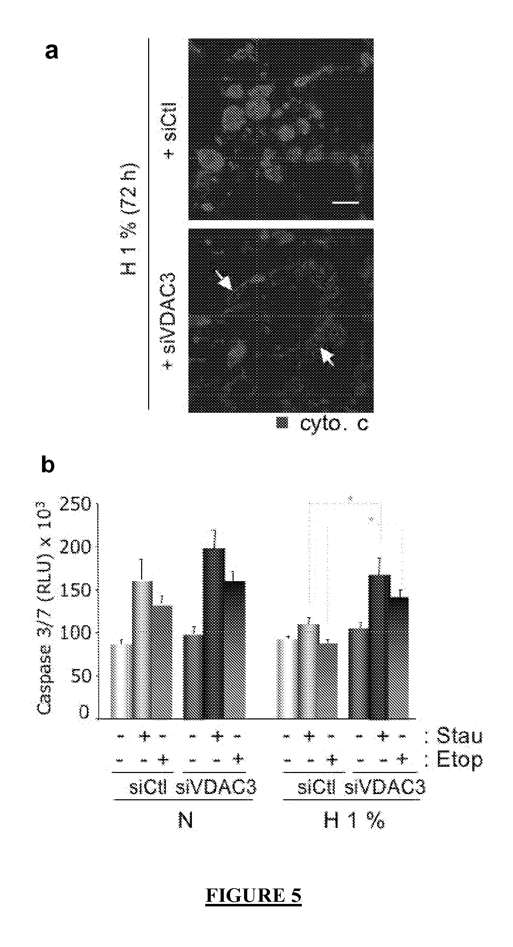

Figure 5 show that hypoxic induction of VDAC3 protects LS174 cells from apoptotic stimuli. Figure 5a shows immunofluorescence of cytochrome c of control (+siCTRL) and VDAC3 silenced (+siVDAC3) LS174 cells. Arrows indicate fragmented mitochondria; bar 7.3 µm. Figure 5b illustrates caspase activity of control (siCTRL) and VDAC3 silenced (siVDAC3) LS174 cells in presence or absence of apoptosis inducing agent staurosporine (Stau, 1 µM) or etoposide 10 µg/ml (Etop) (n=8, 2 experiments, *P < 0.001).

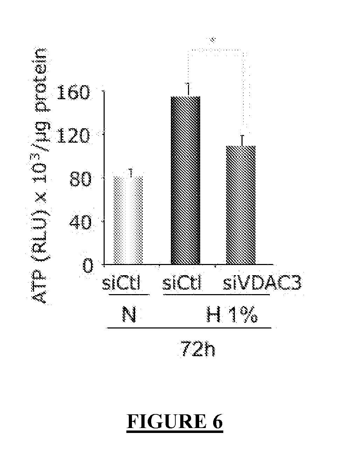

Figure 6 illustrates ATP production of LS174 cells silenced (siVDAC3) or not (siCt1) for VDAC3 in normoxia (N, 21% O₂) or hypoxia (H, 1% O₂) for 72 hours. (n=8, 2 experiments *P < 0.001).

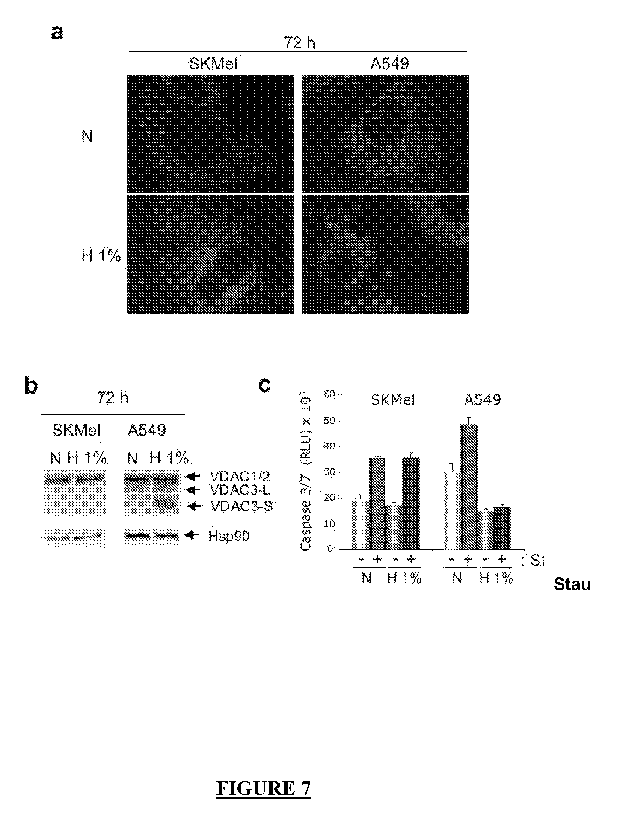

Figure 7 shows that VDAC3-S is induced in apoptosis resistance A549 cells but not in SkMel apoptosis sensitive cells. Figure 7a shows immunofluorescence of cytochrome c in A549 and SkMel cells at 72h after hypoxia (H, 1% O₂) or normoxia (N, 21 % O₂). Figure 7b shows an immunoblot of VADC proteins expressed by A549 and SkMel cells in normoxia (N, 21 % O₂) or hypoxia (H, 1% O₂) after 72 h. Hsp90 was used as loading control. Figure 7c illustrates the caspase 3/7 activity of A549 and SkMel cells in normoxia (N) or hypoxia (H) at 72h without (-) or with (+) staurosporine 1µM (Stau).

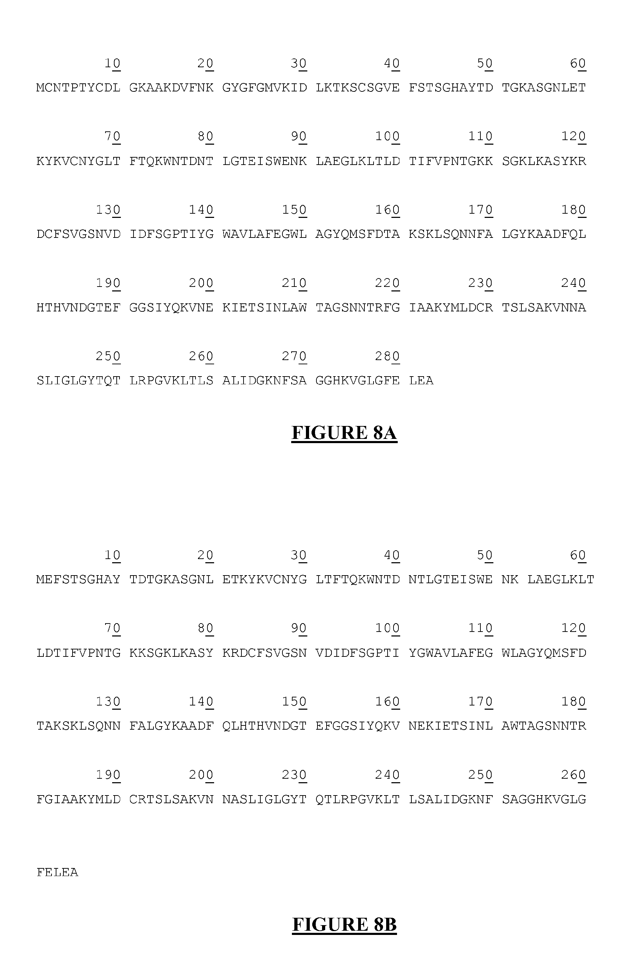

Figures 8A and 8B illustrate polypeptide sequences of VDAC3-L (Fig. 8A) and VDAC3-S (Fig. 8B).

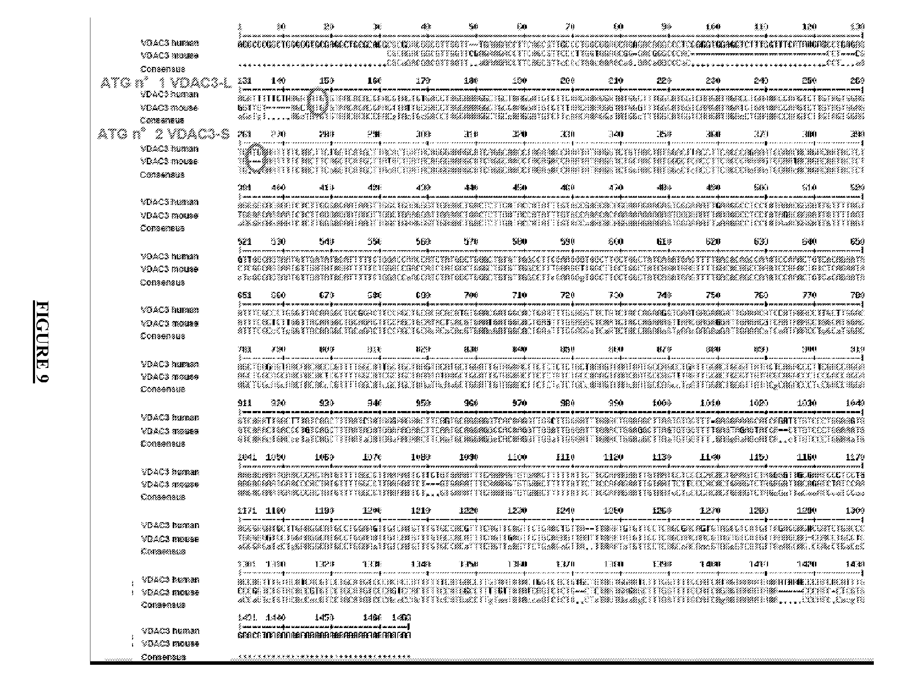

Figure 9 illustrates the polynucleotide alignments of human and mouse VDAC3. Circled codons indicate, respectively, the starting ATG of human VDAC3-L and VDAC3-S.

### EXAMPLES

### METHODS SUMMARY

LS174, PC3, SKMel and A549 cells were grown in Dulbecco's Modified Eagle's Medium (DMEM) (Gibco-BRL) supplemented with 5 or 10% inactivated fetal bovine serum (FBS) as appropriate in penicillin G (50 U/ml) and streptomycin sulphate (50 µg/ml). Dr van de Wetering (van de Wetering *et al.,* EMBO Rep. 2003, 4: 609) kindly provided the LS 174 cells expressing the tetracycling repressor. Stable LS 174 pTerHIF - 1α cells (Chiche et al., Cancer Res 2009, 69 : 358) were incubated without (- Tet) or with (+ Tet) Tet for 4 days to silence hif -1α and maintained in either normoxia 21 % O₂ (N) or hypoxia 1% O₂ (H 1%) for 72h.

For the clonogenicity assay, cells (5000 cells) were seeded in 100 mm dishes and once attached incubated for 72 h in normoxia (N) or hypoxia 1% O₂ (H 1 %) prior to addition or not of staurosporine (Stau) at 0.3 and 0.6 µg/ml. The dishes were then maintained for a further 10 days in normoxia or hypoxia before staining for viable cell colonies.

For hypoxic culture conditions, a Bug-Box™ anaerobic workstation (Ruskinn Technology Biotrace International Plc) set at 1% oxygen, 94% nitrogen and 5% carbon dioxide was used. According to the assay performed, hypoxia was maintained for 24 to 72 hours before the assays.

For microscopy, fixed cells were embedded in epoxy resin, processed for thin sectioning and examined with a Philips CM12 transmission electron microscope equipped with an Olympus SIS CCD camera.

For immunofluorescence microscopy, cells were grown on glass coverslips and then fixed in 3.3% paraformaldehyde for 30 min at room temperature (RT), permeabilized with 0.2% Triton X-100 for 5 min. Cells were then blocked with phosphate-buffered saline (PBS) containing 0.2% gelatin, 2% bovine serum albumin for 30 min at RT, and incubated with anti-mouse cytochrome c (1/500) or anti-rabbit VDAC (1/400) antibodies in PBS for 3 h at RT. After washing, cells were incubated in the presence of a biotinylated anti-mouse secondary antibody conjugated to Alexa 594 (1:400) or anti-rabbit secondary antibody conjugated to Alexa 488 (1:400) for 1 h at RT. After washing, coverslips were mounted in Cytifluor (Amersham Biosciences), detection of the fluorescence was performed with a Leica DMR fluorescence microscope, and images were recorded using RSImage software.

For electron microscopy, cells were fixed in situ with 1.6% glutaraldehyde in 0.1 M phosphate buffer at RT and stored overnight at 4°C. Samples were rinsed in the same buffer and then post-fixed with 1% osmium tetroxide and 1% potassium ferrocyanide in 0.1 M cacodylate buffer for 1 h at RT to enhance the staining of cytoplasmic membranes (de Bruijn, 1973, Ultrastruct. Res., 42:29). Cells were rinsed with distilled water, embedded in epoxy resin, sectioned and examined with a Philips CM12 transmission electron microscope equipped with an Olympus SIS CCD camera. The area of mitochondria of cells was calculated as an ellipse (0.785 x a x b) (n=2, 50 mitochondria per experiment) (mean ± s.e.m.).

For transfection of siRNA, siRNA sequences targeting SIMA (named sictl) has been described previously (Berra et al., EMBO J., 2003, 22: 4082; ). siRNA sequences targeting VDAC3 were purchased from Santa Cruz Biotechnology (VDAC3 siRNA (m): sc-42360). Cells were transfected with 40 nM of a control siRNA (sict1) or siRNA to invalidate VDAC3, 24 h prior to normoxic or hypoxic treatment (1 % 02).

For ATP determination, PC3 and LS174 cells were incubated first in hypoxia for 72 h (H 1% 72 h) then either lysed or cultured in normoxia for 24 h in the absence (-) or presence (+) of glucose or the inhibitor of mitochondrial respiration oligomycin. Quantification of ATP was done using a luciferin/luciferase-based assay (Cell Titer Glo kit, Promega) according to the manufacture's instructions and results are expressed as relative luminescence units (RLU). Each condition was tested 8 times and the entire experiment was done twice. Significant differences are based on the Student's t test *** P < 0.005.

For caspase activation, quantification of the caspase 3/7 activity was done using a luciferin/luciferase based assay (Caspase-Glo 3/7 kit, Promega) according to the manufacture's instructions. Each condition was performed 8 times and the entire experiment was done 3 times. Significant differences are based on the Student's t test * P < 0.005. Staurosporine was added (+ Stau) 4 h prior to assay for caspase 3/7 activity.

For the flow cytometry, the following fluorochromes were employed to determine mitochondrial function by cytofluorometry: 3,3'-dihexyloxacarbocyanine iodide [DiOC6(3), 50 nM] for mitochondrial transmembrane potential (ΔΨm) quantification and propidium iodide (PI; 1 µg/ml) for determination of cell viability (all from Molecular Probes). Cells were trypsinized and labeled with the fluorochromes at 37°C, followed by cytofluorometric analysis with a fluorescence-activated cell sorter (FACS) scan (Becton Dickinson). Cells cultured in normoxia were incubated with or without a mitochondrial decoupling compound (CLCCP) for 15 min prior to addition of DiOC (50 µM) and propidium iodide (50 µg/ml). The fluorescence intensity of DiOC6 (3) (which measures ΔΨm) is plotted against the cell size (forward scatter).

For the RNA extraction, PCR and relative quantitative PCR, the total RNA was extracted from cells using the RNA extraction kit (Qiagen) according to the manufacturer's instructions. Total RNA (2 µg) was added to a 20 µl reverse transcription-PCR (RT-PCR) reaction, using the Omniscript kit (Qiagen). Semi-Q RT-PCR was performed using the Taq PCR Mastermix kit (Qiagen). Two pairs of oligonucleotides (sense primer ATG n°1, 5'-GGCATGGTCAAGATAGACCTG-3'; sense primer ATG n°2, 5'-ATGGAATTTTCTACTTCTGGTC-3'; and a common antisense primer, 5'-GTGTAAGCATGACCAGAAGTAG-3'; 94°C for 30 sec, 55°C for 1 min and 72 °C for 1 min, 30 cycles) were designed to amplify VDAC3-L and VDAC3-S isoform. 36B4 was used as an internal control for normalization. PCR products were size-separated on a 1.5% agarose gel, treated with ethidium bromide staining and visualized using electrophoresis image analysis system.

For immunoblotting, cells were lysed in 1.5 x Laemmli buffer and the protein concentration determined using the BCA assay. 40 µg of protein of whole cell extracts was resolved by SDS-PAGE and transferred onto a PVDF membrane (Millipore). Membranes were blocked in 5% non-fat milk in TN buffer (50 mM Tris-HCl pH 7.4, 150 mM NaCl) and incubated in the presence of the primary and then secondary antibodies in 5% non-fat milk in TN buffer. After washing in TN buffer containing 1% Triton-X100 and then in TN buffer, immunoreactive bands were visualized with the ECL system (Amersham Biosciences).

Regarding statistics, all values are the means ± s.d. of the indicate number of determinations (n) and significant differences are based on the Student's *t* test and P values indicated.

### Example 1

### Formation of enlarged mitochondria in hypoxia

PC3 (prostate carcinoma) cells exposed to hypoxia conditions for 72 hours had tubular mitochondria (Fig. 1a, *left*) while LS 174 (colon carcinoma) cells showed enlarged mitochondria and cristae with an intact membrane ultrastructure (Fig. 1a, *right panel*; Fig. 1b). The contrast of the mitochondrial matrix of LS174 cells on the electron micrographs was maintained, which suggested the absence of mitochondrial swelling. In addition, the surface area of mitochondria of PC3 cells detected by electron microscopy remained constant for increasing periods of hypoxia (1% 02) while that of LSI74 cells increased and was substantially higher at 72 h of hypoxia (Fig. 1c).

### Example 2

### Enlarged mitochondria are functional

The mitochondrial transmembrane potential (ΔΨm), measured by accumulation of DiOC₆(3) (Kim et al., Cell Metab, 2006, 3:177), was not modified in hypoxia in both PC3 and LS174Tr cells in which hif-1α was silenced by tetracyline-(Tet) inducible shRNA expression (Chiche et al., Cancer Res, 2009, 63: 358) while a decrease in the ΔΨm occurred when cells were treated with the mitochondrial decoupling agent (CLCCP) (Fig.2a). The level of ATP for PC3 and LS174Tr cells in hypoxia was also the same (Fig. 2b). When hypoxic cells were subsequently reoxygenated in fresh medium containing glucose (+) but in the absence (-) or the presence (+) of the inhibitor of mitochondrial respiration, oligomycin, more ATP was produced. In the absence of glucose (-) a decrease in ATP production that was sensitive to oligomycin in both cell lines was observed.

These results indicate that both cell lines participate in glycolysis and mitochondrial respiration for glucose metabolism and ATP production and thereby show that enlarged mitochondria, like tubular mitochondria, are functional.

### Example 3

### Cells with enlarged mitochondria are protected from apoptotic stimuli

To test the response of hypoxic LS174 cells to apoptosis, these cells were treated with staurosporine for 24 h or 72h (1 µM) (Fig. 3a) or with etoposide for 24h (10 µg/ml) (Fig. 3b), known inducers of mitochondria-dependant apoptosis.

Staurosporine (Stau) increased the caspase 3/7 activity, an indicator of apoptosis (Fig. 4a), and increased the number of apoptotic nucleic in cells exposed to normoxia and hypoxia for 24h. However, after prolonged incubation in hypoxia (72h) LS174 cells resisted staurosporine. Similar results were observed in the presence of etoposide (Etop), a topoisomerase II inhibitor used in cancer therapy (Fig. 3b).

Hypoxic LS174 cells resisted a high concentration of staurosporine (1µM) for at least 24 h or for 10 days at a lower concentration (0.3 or 0.6 µg/ml (Fig. 3c).

Thus, hypoxic pretreatment provides long-term protection against staurosporine. However, this protection was reversible since reoxygenation for 48h in the presence of staurosporine restored sensitivity to staurosporine-induced apoptosis.

It is concluded here that enlarged mitochondria are insensitive and thus protected cells from apoptotic stimuli in a hypoxic environment.

### Example 4

### Hypoxia VDAC3 induction blocks apoptosis

The decrease in the expression of a variety of proteins potentially involved in the control of cytochrome c release from mitochondria (Fig. 4a), a key feature of apoptosis, was compared, at 24 and 72 h of hypoxia, times at which LS174 cells are sensitive or resistant to staurosporine-induced apoptosis, respectively.

Bak and VDAC3 were induced at 72 h of hypoxia (Fig. 4a). Bcl-X_{L} was only slightly, but reproducible, induced in LS174 cells.

Thus, increased expression of Bak, VDAC3 and Bcl-X_{L} correlated with the presence of enlarged mitochondria and resistance to an apoptotic stimulus.

Hypoxic induction of one VDAC3 protein isoform was noted in resistant hypoxic LS 174 cells (Fig. 4a), but not in hypoxic PC3 cells (Fig. 4a) which are sensitive to apoptosis.

Using two set of different primers, it was demonstrated that the long form of VDAC3 (VDAC3-L) is not induced in hypoxia whereas the shorter isoform of VDAC3 (VDAC3-S) is induced under hypoxic conditions (Fig. 4c).

Silencing of VDAC3 (L and S) by RNA interference in LS174 cells (Fig. 4b) leads to a decrease in the number of enlarged mitochondria (Fig. 5a) and partially reestablished the sensitivity of hypoxic cells to staurosporine and to etoposide (Fig. 5b). Cells silenced for VDAC3 also produced less ATP in hypoxia (Fig. 6).

### Example 5

To verify that the protection from apoptosis of cells with enlarged mitochondria and enhanced expression of VDAC3-S was not restricted to the LS174 cell line, two other cell lines A549 and SkMel cells that show enlarged or tubular mitochondria, respectively (Fig. 7a) were tested for their hypoxic VDAC3 expression. VADC3 expression was strongly induced in A549 cells but totally absent in SkMel cells (Fig. 7b). Moreover, hypoxic A549 cells resisted a high concentration of staurosporine whereas hypoxic SkMel cells were sensitive (Fig. 7c).

Taken together, these results demonstrate that VDAC3-S participates in mitochondrial protection against apoptosis of hypoxic cells exposed to an apoptotic stimulus.

## Claims

1. Use of VDAC3-S as a cell marker of apoptosis-resistance phenotype of hypoxic cancer cells.

2. The use according to the preceding claim, wherein a presence or an increased level of expression and/or activity of VDAC3-S is indicative of an apoptosis-resistance phenotype.

3. The use according to claim 1 or 2, wherein the cell marker is a polynucleotide or a polypeptide.

4. The use according to anyone of the preceding claim, wherein the cell marker is represented by a polynucleotide encoding a polypeptide as set forth in SEQ ID NO: 2, an analogue thereof having an identity of sequence of at least 90%, or a fragment thereof encoding for a polypeptide having at least 8 contiguous amino acids of a polypeptide as set forth in SEQ ID NO: 2.

5. The use according to anyone of claim 1 to 3, wherein the cell marker is represented by a polypeptide as set forth in SEQ ID NO: 2, an analogue thereof having identity of sequence of at least 90% and a similar biology activity, or a fragment thereof having at least 8 contiguous amino acids of a polypeptide as set forth in SEQ ID NO: 2.

6. The use according to anyone of the preceding claims wherein a presence, or an increased of level of expression and/or activity of VDAC3-S is associated with a presence mitochondria of enlarged phenotype.

7. A method for determining an apoptosis-resistance phenotype of at least one isolated hypoxic cancer cell suspected to be apoptosis-resistant comprising at least the steps of:
a) determining in said hypoxic cancer cell a presence or an absence, or a level of expression and/or activity of VDAC3-S, and
b) comparing said determined presence or absence, or level of expression and/or activity to a control value or range.

8. The method according to the preceding claim, wherein step a) is carried out by a method chosen from mass spectrometry, chromatographic separations, binding assays, enzyme assays, or competitive inhibition assays

9. The method according to claim 7 or 8, wherein step a) is carried out by a method chosen from Western-blot, immunoassays, ELISA, fluorescence microscopy, flow cytometry, direct sequencing, gel electrophoresis, column chromatography, PCR, RT-PCR, or quantitative PCR.

10. The method according to anyone of claims 7 to 9 wherein a presence, or an increase of said level of expression and/or activity relative to the control value or range detects an apoptosis-resistance phenotype.

11. The method according to anyone of claims 7 to 10, comprising further a step of determining an absence or a presence of mitochondria of enlarged phenotype.

12. A method for assessing a resistance of a cancer to a cancer treatment in a subject, the method comprising at least a step of comparing a presence or an absence, or a level of expression and/or activity of VDAC3-S in a first biological sample obtained from the subject before the treatment has been administered to the subject, and the presence or absence, or level of expression and/or activity of VDAC3-S in a second biological sample obtained from the subject after the treatment has been administered to the subject, wherein a change in the presence or absence, or level of expression and/or activity of VDAC3-S in the second sample relative to the first sample is an indication on the resistance of a cancer to said cancer treatment.

13. A method for screening candidate active agents able to restore apoptosis-sensitivity to an apoptosis-resistant hypoxic cancer cell comprising at least the steps of:
a) providing at least one isolated cell able to express VDAC3-S or a non-human transgenic animal able to express VDAC3-S in conditions suitable for the expression of VDAC3-S,
b) contacting said isolated cell or said non-human transgenic animal with at least one candidate active agent to be screened,
c) determining in said isolated cell or in said non-human transgenic animal a presence or absence, or a level of expression and/or activity of VDAC3-S, and
d) comparing said determined presence or absence, or level of expression and/or activity to a control value or range.

14. A method for screening candidate active anticancer agents comprising at the least the steps of:
a) subjecting at least one candidate active anticancer agent known as having anticancer activity to the method as defined in claim 13, and
b) selecting the candidate active anticancer agent able to reduce, suppress and/or prevent a presence, or an expression and/or an activity of VDAC3-S.

15. Use of at least one nucleic acid sequence able to reduce and/or suppress and/or prevent the presence, or the level expression and/or activity of VDAC3-S for manufacturing a pharmaceutical composition for restoring sensitivity of an hypoxic apoptosis-resistant cancer cell to an apoptosis-inducing agent for the treatment of cancer.

16. The use according to the preceding claim, wherein the nucleic acid sequence is a siRNA, a miRNA, a shRNA, an hybrid sequence, or a synthetic or semi-synthetic sequence of oligonucleotide.

17. The use according to claim 15 or 16, wherein the cancer is chosen from breast cancer, head-and-neck cancer, liver cancer, ovarian cancer, pancreatic cancer, prostate cancer, renal cancer, skin cancer, testis cancer, lung cancer, cervical cancer or brain cancer.

18. Use of mitochondria of enlarged phenotype as a cell marker of apoptosis-resistance phenotype of hypoxic cancer cells.


SEQUENCE LISTING

<110>  Centre National de la Recherche Scientifique

<120>  VDAC3-S AS A CELL MARKER

<130>  BR88154

<160>  2

<170>  PatentIn version 3.3

<210>  1
<211>  283
<212>  PRT
<213>  Homo Sapiens

<400>  1

Met Cys Asn Thr Pro Thr Tyr Cys Asp Leu Gly Lys Ala Ala Lys Asp
1               5                   10                  15

Val Phe Asn Lys Gly Tyr Gly Phe Gly Met Val Lys Ile Asp Leu Lys
            20                  25                  30

Thr Lys Ser Cys Ser Gly Val Glu Phe Ser Thr Ser Gly His Ala Tyr
        35                  40                  45

Thr Asp Thr Gly Lys Ala Ser Gly Asn Leu Glu Thr Lys Tyr Lys Val
    50                  55                  60

Cys Asn Tyr Gly Leu Thr Phe Thr Gln Lys Trp Asn Thr Asp Asn Thr
65                  70                  75                  80

Leu Gly Thr Glu Ile Ser Trp Glu Asn Lys Leu Ala Glu Gly Leu Lys
                85                  90                  95

Leu Thr Leu Asp Thr Ile Phe Val Pro Asn Thr Gly Lys Lys Ser Gly
            100                 105                 110

Lys Leu Lys Ala Ser Tyr Lys Arg Asp Cys Phe Ser Val Gly Ser Asn
        115                 120                 125

Val Asp Ile Asp Phe Ser Gly Pro Thr Ile Tyr Gly Trp Ala Val Leu
    130                 135                 140

Ala Phe Glu Gly Trp Leu Ala Gly Tyr Gln Met Ser Phe Asp Thr Ala
145                 150                 155                 160

Lys Ser Lys Leu Ser Gln Asn Asn Phe Ala Leu Gly Tyr Lys Ala Ala
                165                 170                 175

Asp Phe Gln Leu His Thr His Val Asn Asp Gly Thr Glu Phe Gly Gly
            180                 185                 190

Ser Ile Tyr Gln Lys Val Asn Glu Lys Ile Glu Thr Ser Ile Asn Leu
        195                 200                 205

Ala Trp Thr Ala Gly Ser Asn Asn Thr Arg Phe Gly Ile Ala Ala Lys
    210                 215                 220

Tyr Met Leu Asp Cys Arg Thr Ser Leu Ser Ala Lys Val Asn Asn Ala
225                 230                 235                 240

Ser Leu Ile Gly Leu Gly Tyr Thr Gln Thr Leu Arg Pro Gly Val Lys
                245                 250                 255

Leu Thr Leu Ser Ala Leu Ile Asp Gly Lys Asn Phe Ser Ala Gly Gly
            260                 265                 270

His Lys Val Gly Leu Gly Phe Glu Leu Glu Ala
        275                 280

<210>  2
<211>  245
<212>  PRT
<213>  Homo Sapiens

<400>  2